Europäisches Patentamt

European Patent Office

Office européen des brevets

(19)

(11) Publication number: 0 262 758
A1

# (12) EUROPEAN PATENT APPLICATION

(21) Application number: 87305823.4

(22) Date of filing: 01.07.87

(51) Int. Cl.⁴: **A61F 2/64**

(30) Priority: 07.08.86 GB 8619298

(43) Date of publication of application:
06.04.88 Bulletin 88/14

(84) Designated Contracting States:
DE FR

(71) Applicant: **J.E. HANGER & COMPANY LIMITED**
**Roehampton Lane**
**Roehampton London SW15 5PL(GB)**

(72) Inventor: **Cooper, John Edwin**
**St. Gallen The Ballands North**
**Leatherhead KT22 9HU(GB)**

(74) Representative: **Cole, Paul Gilbert et al**
**Hughes Clark Andrews & Byrne 63 Lincoln's**
**Inn Fields**
**London WC2A 3JU(GB)**

(54) **Improvements in knee prosthesis.**

(57) A knee prosthesis comprises a top hinge member(10), a bottom hinge member (12) having spaced cheek plates (36) facing in a posterior direction, pivot means (14) interconnecting the top and bottom hinge member (10, 12), control rod means (22) depending from and pivoted at an upper end to the top hinge member (10) and oscillating trunnion means (30) pivoted between the cheek plates (36). A transverse aperture (46) in which the control rod means (22) is slideably received is formed in the trunnion means (30) and means (54, 56) operatively connecting the control rod means (22)and the trunnion means (30) arrests relative movement between the top and bottom hinge members. A range of control functions for the prosthesis are described including a spring assist (52) that biases the knee towards its flexed and towards its extended positions, a knee lock, a damper that controls extension of the knee during the swing phase of walking and actuating member operates clutch means in the lower hinge member (12) when the weight of the user is applied.

FIG. 1

FIG. 2

## IMPROVEMENTS IN KNEE PROSTHESIS

This invention relates to a knee prosthesis.

It is an object of the invention to provide a family of controls for a knee prosthesis that can be added to the basic limb and that uses, so far as is possible, common or compatible components.

The invention provides a knee prosthesis comprising a top hinge member, a bottom hinge member having spaced cheek plates facing in a posterior direction, pivot means interconnecting the top and bottom hinge member, control rod means depending from and pivoted at an upper end to the top hinge member, oscillating trunnion means pivoted between the cheek plates and having a transverse aperture in which the control rod means is slideably received, and means operatively connecting the control rod means and the trunnion means to arrest relative movement between the top and bottom hinge members.

The control rod means may be formed with a collar located between its pivoted upper end and the trunnion means in which case a coil spring on the control rod means and held in compression between the collar and the trunnion means urges the top and bottom hinge members towards their extended positions at low angles of flexion. Preferably the pivoted upper end of the control rod means is arranged to pass from posterior to anterior side of a line joining the knee pivot means with the trunnion means so that the top and bottom hinge members are urged to an extended position at high angles of flexion.

The prosthesis may include knee lock means operable to arrest relative movement of the top hinge member and the bottom hinge member at an unflexed attitude of the knee, said knee lock means being operatively connected to the control rod means. A preferred knee lock means is a latch bar rotatable about a transverse axis to and from an operative position between a nose on the control rod means and a longitudinally slotted member on the trunnion means, the latch bar when in its operative position preventing the nose from descending along the slot so that the knee is held unflexed. A packing piece of adjustable length may be provided between the slotted member and the trunnion means so that the hyperextension angle can be varied without introducing backlash.

Damper means may be operably connected to the control rod means to control extension of the knee during the swing phase of walking and may be arranged to offer minimal resistance during flexion of the knee, the damper means may comprise a cylinder, an annular piston in the cylinder, tubular piston rod means in the cylinder that passes through the piston and through a lower end of the cylinder to contact abutment means at a lower end of the control rod means and movable therewith, means urging the piston rod means against the abutment means, lost motion connector means interconnecting the piston rod means and the piston so that when the piston rod descends as the knee flexes the piston is spaced from a collar on the piston rod means and fluid can flow directly from the piston to the rod side of the cylinder and when the piston rod ascends as the knee unflexes direct fluid flow from the rod to the piston side of the cylinder is prevented, by-pass passage means including a flow restricting orifice interconnecting rod and piston sides of the cylidner to provide a fluid return path.

In a further variant of the knee prosthesis means in the top hinge member acting on the control rod means locks the position of the control rod when the weight of the user is applied to the leg thereby arresting flexion upper parts of the limb may be hinged to the top hinge member for rotation about a transverse axis, an actuating member attached to the platform applying clutch means in the lower hinge member when the weight of the user is applied and the actuating member may be a leaf spring. The clutch may be a roller having a transversely directed axis captive between an anterior wall on the trunnion means and a sliding plate defined by a transversely enlarged region of the control means, said plate and said anterior wall defining between them a space of downwardly convergent profile in side view.

Various embodiments of the invention will now be described, by way of example only, with reference to the accompanying drawings, in which:-

Figure 1 is a side elevation of a first knee prosthesis that has provision for adjustable hyperextension and extension assist;

Figure 2 is a transverse section of the knee of Figure 1 on the line A-A;

Figure 3 is a side elevation of a second knee prosthesis that has provision for locking the knee in an unflexed position in addition to adjustable hyperextension and extension assist;

Figure 4 is a front elevation of a locking mechanism forming part of the prosthesis of Figure 3;

Figures 5 and 6 are a front and side elevation respectively of an extension rod forming part of the locking mechanism of the knee of Figure 3;

Figures 7 and 8 are a front and side elevation respectively of latch member forming part of the locking mechanism of the knee of Figure 3;

Figure 9 is a side elevation of a third knee prosthesis providing for extension control during the swing phase of walking in addition to provision for knee lock, extension assist and adjustable hyperextension;

Figure 10 is a transverse section of the knee on the line B-B of Figure 9;

Figure 11 is a side elevation of a fourth knee prosthesis providing a so-called "Stabilised" knee that locks under the user's weight during standing; and

Figure 12 is a transverse section of the knee of Figure 11.

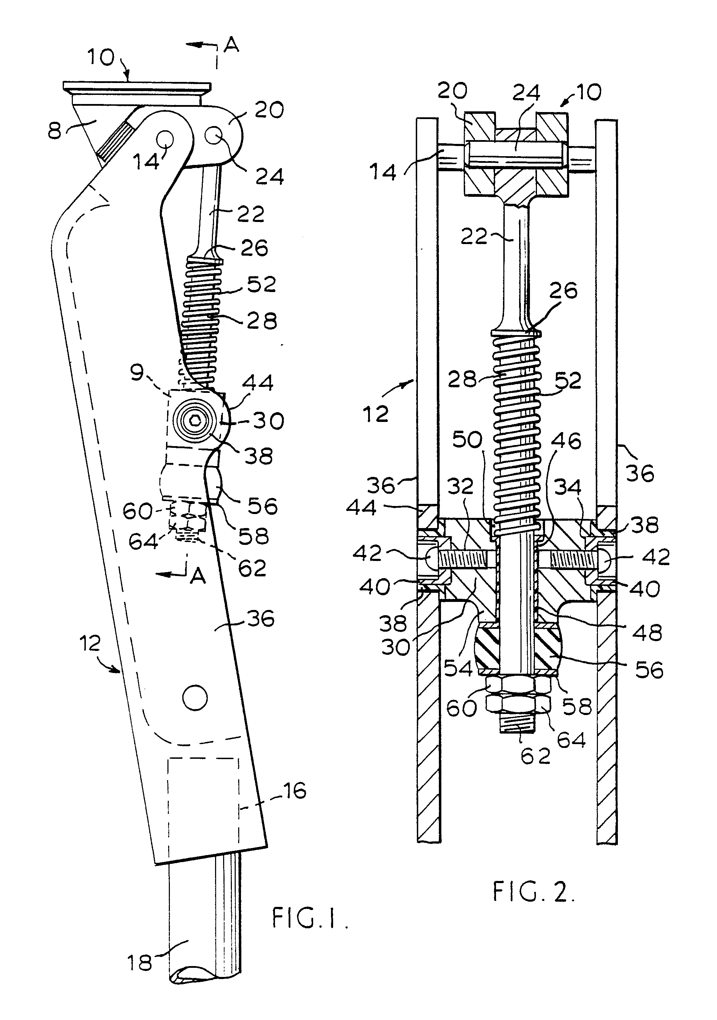

In Figures 1 and 2 of the drawings, a top hinge 10 is connected to a shin body 12 by means of a pivot pin 14. The shin body 12 is generally of rearward facing channel section and terminates in a socket 16 that fits over a top end of a shin tube 18. A nose piece 8 is at a clearance from the underlying regions of the shin body 12 in the illustrated normally extended position and permits up to 10° hyperextension before the nose piece 8 interferes with the shin body. The top hinge 10 has a rearwardly facing bifurcated bracket 20 between the limbs of which the upper end of an extension rod 22 is pinned by means of a pin 24. The rod 22 is formed with a collar 26 and a depending shank 28 that fits in and slides through a central hole in a trunnion 30.

The trunnion 30 has a central longitudinal through hole whose ends are internally threaded at 32 and open at enlarged end face recesses 34. Cheek plates 36 of the shin body 12 have flanged bearing sleeves 38 of PTFE Delrin or other suitable material that are push fitted into apertures in the plates 36 from the inner faces thereof. Stub axles 40 are inserted from the outer faces of cheeks 36 into the sleeves 38 and locate in the recesses 34 of the trunnion 30. Retaining bolts 42 are received in threaded regions 32 of the through hole to hold the stub axles in place. The stub axles 40 rotate in the sleeves 38 with the trunnion 30 as the knee flexes and unflexes and the extension rod 22 causes the trunnion 30 to oscillate. The trunnion 30 is pivoted in ear formations 44 of the plates 36 at an intermediate position along the shin body 12.

A transverse through hole 46 in the trunnion 30 is lined with a self-lubricating plastics bearing sleeve 48 of Delrin, PTFE or other suitable material that is a friction fit therein. An upper portion of the hole 46 through trunnion 9 is enlarged at 50 to provide a seat for a lower end of a coil spring 52 that fits onto the shank 28 and is held in compression by abutment of its upper end with the collar 26. The trunnion 30 is formed with a depending spigot 54 and the lower end of shank 28 protrudes from spigot 34. An annular buffer washer 56 of resilient material is bonded to a metal washer 58 that is preferably attached to an adjustment nut 60 that is received on a threaded lower region 62 of the shank 28, through the washer 58 and nut 60 may be free of so desired. Abutment of the buffer 56 with the spigot 54 limits extension of the top hinge 10 and shin body 12 and the actual hyperextension angle can be set by rotating the adjustment nut 60. Because the spigot 54 is impacted by elastomeric buffer 56 as the extended position is reached there is substantially no sound as this occurs. The desired position of nut 60 is preserved by locknut 64.

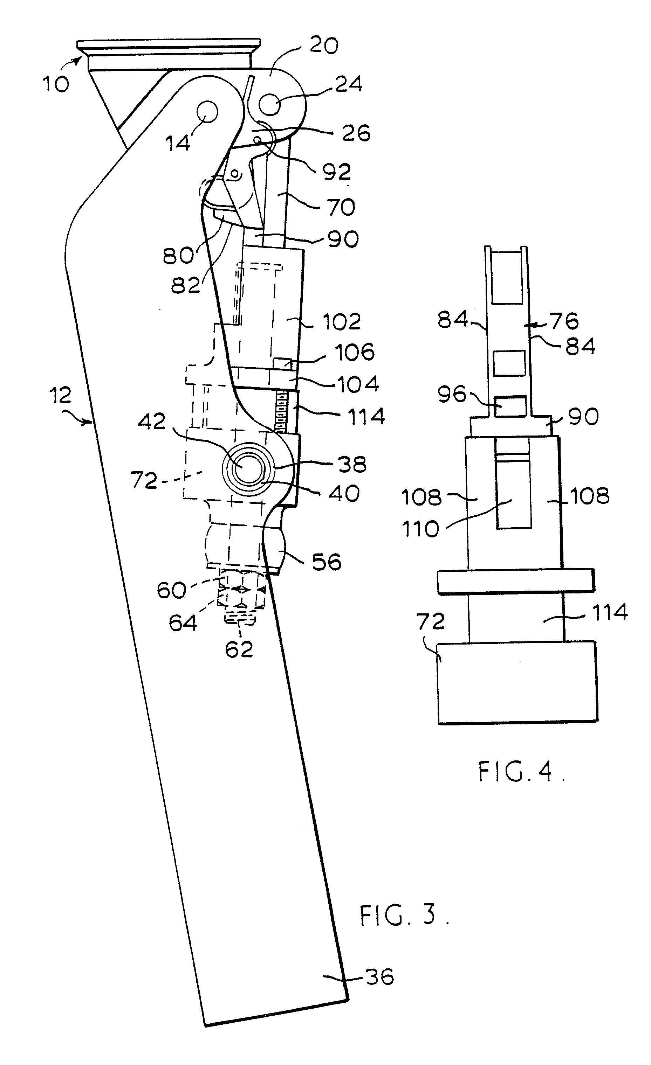

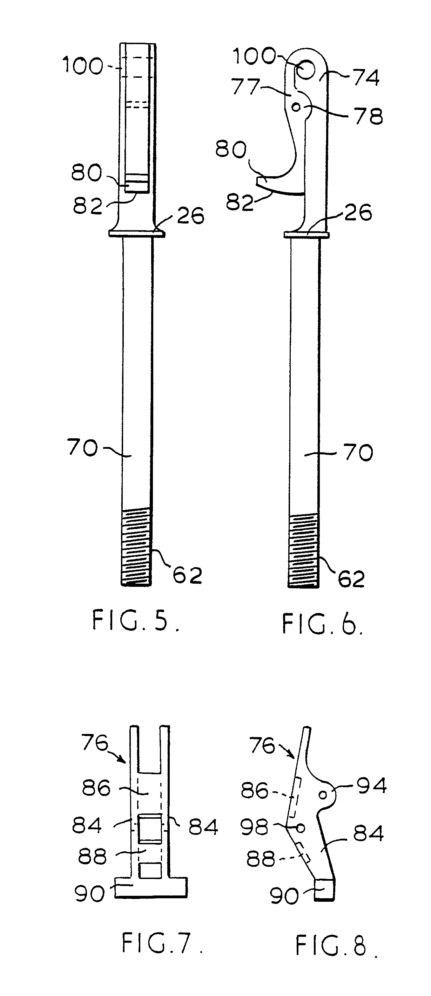

Figures 3 and 4 show a second embodiment of the invention in which there is a knee lock associated with the extension rod and hyperextension adjustment mechanism. An extension rod 70 is pivoted at 24 to the bracket structure 20 of the upper hinge member 10 and passes through a trunnion 72 as before. The rod 70 as shown in Figures 5 and 6 has a enlarged head structure above the collar 26, which is formed with lateral flats 74 that pass between the limbs of bracket structure 20. Anterior regions 77 of the lateral faces of the rod 20 are relieved to receive a latch member 76 which is connected thereto at pivot region 78. Below region 78 is provided an anterior facing nose 80 having a curvilinear lower face 82, and the region between face 82 and collar 26 is flat for reception of a latch bar (described below). The latch member 76 is formed in one piece and includes side plates 84 and upper, intermediate and lower cross-members 86, 88, 90, the cross-member 90 being extended laterally to define a latch bar. The cheek plates 84 are slideably received on the anterior regions 77 of the extension rod 70 to which they are pivoted by means of a pin 92 that passes through apertured prosterior facing ears 94 and through aperture 96 in the extension rod 70. Accordingly the latch member 76 normallly hangs down in contact with the rod 70 but can pivot through a small angle in an anterior direction, the nose 80 passing through an aperture 96 between the cross-member 88 and latch bar 90. The plates 84 are formed with apertures 98 to receive the lower end of a leaf spring (not shown) which passes under cross-member 86 with its upper end held in compression against the anterior face of the extension rod 70 adjacent aperture 100 for the pin 24. Rotation of the latch member 76 in the anterior direction increases the compression of the leaf spring, which thereby biases the member 76 towards the rod 70.

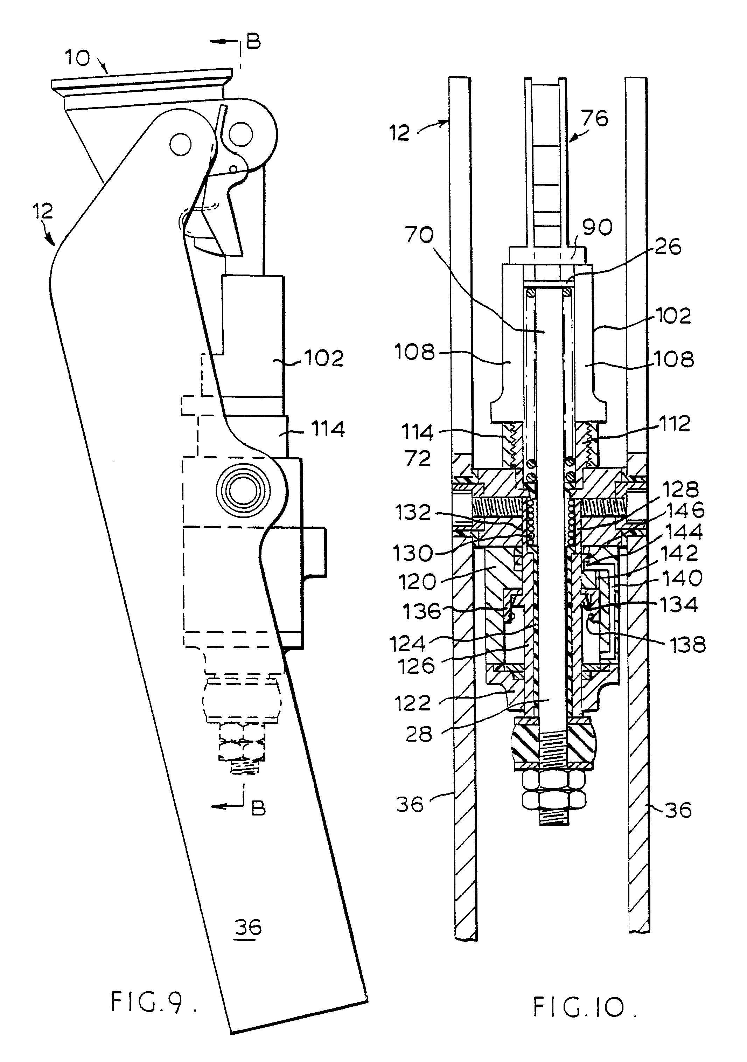

Between the locking bar 90 and the trunnion 72 are fitted an extension rod sleeve 102 and an adjustable spacer member. The sleeve 102 is formed with a pedestal 104 that is triangular in plan and apertured to receive three fixing bolts 106 that pass around the spacer member and are received in threaded bores in the top face of the trunnion 72. The sleeve 102 has a cut-away anterior portion defining lateral bearing surfaces 108 and a central slot 110, the internal diameter of the sleeve being such that the collar 26 and coil spring 52 are a close fit therein. The slot 110 is wide enough that the nose 80 can enter it and move downwardly as the knee flexes. The latch bar 90 in flexed positions of the knee will have been rotated in an anterior direction and slides over the faces 108. When the knee is unflexed it passes off the faces 108 and is snapped by the leaf spring into the gap between the nose 80 and the top face of the sleeve 102 to take up a locking position shown in Figure 3. The knee now cannot flex until the latch member 76 has been rotated sufficiently to align the locking bar with the faces 108, this being achieved by means of a release cable (not shown) operatively connected to member 76. Provision is made for locking the release cable to hold the latch bar 90 in its disengaged anterior position thereby allowing the knee to flex freely during ordinary walking. The height of the sleeve 102 relative to trunnion 72 needs to be adjustable to take account of the variation in hyperextension angle (described above) and avoid introduction of backlash. The adjustable spacer is seen in Figure 10 and comprises an externally threaded inner member 112 that is received as its lower end in a recess in the top face of trunnion 72 and carries a thumb nut 114 around which the bolts 106 pass. Rotation of the thumb nut varies its height and when the proper height has been achieved that latch bar 90 can snap home reliably without backlash the bolts 106 are tightened to bring the sleeve 102 down against thumb nut 114.

Figures 9 and 10 show a further embodiment of the invention in which an extension control cylinder operative in the swing phase of walking to control extension of the shin is fitted on the extension rod 70 below the trunnion 72. A damper cylinder 120 is bolted beneath the trunnion 72 and has at its lower end a cylinder head 122 through which the shank 28 of extension rod 70 passes. The shank 28 enters the cylinder 120 via a flanged sleeve 124 of low friction material that lines a piston rod 126. The bore through rod 126 is stepped at its top end to define an enlarged upper region 128 and the flanged sleeve 124 is inserted from the upper end of the piston rod 126 so that is flange locates on the step as shown. A primary spring 130 fits around the shank 28 within the upper region 128 and abuts against an upper end of a counterbore 132 in the trunnion 72 with which the upper region 128 of rod 126 slides. Accordingly the rod 126 is normally biased downwardly so that it follows the movement of the buffer and adjustment nuts. It has within the cylinder 120 a collar or seat 134 that fits into the skirt of an annular floating piston 136 and is retained by circlip 138 in the skirt of the piston. The internal diameter of the crown of floating piston 136 is such that there is a clearance from the rod 126 and the spacing between the lower face of the crown and circlip 138 is such that there is lost motion between the rod 126 and the collar 134. When the rod 126 is moving upwardly fluid pressure urges the crown of piston 136 onto the collar 134 and fluid cannot pass directly above the piston 136. But when the rod 126 is moving downwardly the piston 136 trails behind the collar 134 so that a gap opens between the crown and the collar 134 through which fluid can pass to the rod side of piston 134, thereby providing a one-way valve mechanism for direct fluid flow between the two sides of the piston 136. Downward motion of rod 126 assisted by primary spring 130 occurs during knee flexion as the rod 70 moves down, during which the damper offers only slight resistance because of direct fluid flow between the two sides of piston 136. But during upward motion of rod 126 against primary spring 130 during knee extension the damper offers resistance because the collar 134 and the crown of piston 136 are now in contact, closing off direct flow between the two sides of piston 136. In order to allow extension to occur the two sides of piston 136 are interconnected by by-pass passage 140 that includes a variable orifice 142 by which a controlled resistance to flow of oil or other working fluid can be set. The passage 140 has a branch 144 leading to a seal 146 between the rod 126 and the cylinder 120 so that passage 140,144 are maintained at low pressure and the seals are maintained unpressurised.

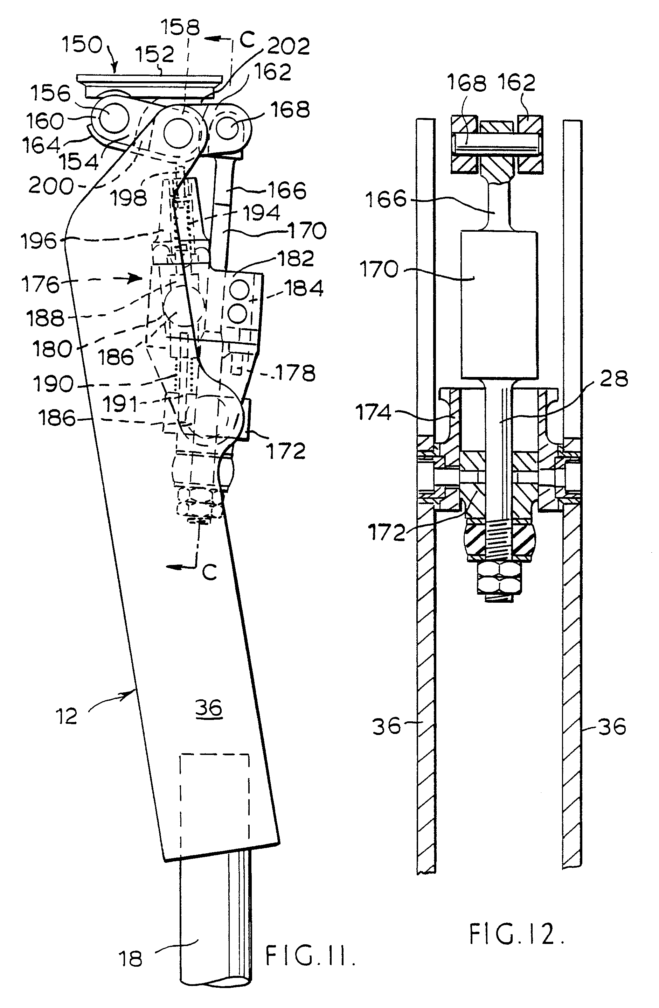

Figures 11 and 12 show a further embodiment of the invention relating to a so-called stabilised knee. An upper member 150 is formed with a platform 152 pivoted above an arm 154. Platform 152 is disc-like in plan with a depending anterior protuberance that is formed with a through-hole for a loading pivot 156. Flat sides of the protuberance fit between anterior bracket member 158 of the arm 154. An anterior portion 160 of the arm 154 is slightly upwardly cranked as shown in order to support the platform 152 at a slight clearance from a posterior portion 162 of the arm 154, so that the platform 152 can rotate about the loading pivot 156 a small distance when the patient's weight is applied. The protuberance of the platform 152 has rigidly attached thereto leaf spring 164 that is profiled similarly to the anterior portion 160 of arm 154 against whose lower face it is positioned. Spring 164 rotates with platform 152 as the patient's weight is applied. The posterior portion 162 is bifurcated to receive the upper end of an extension rod 166 that is pivoted thereto at 168. The rod 166 is plain except for a bearing plate 170 above the shank 28 and passes through a trunnion 172 of plastics or other low friction material that is supported from cheek plates 36 somewhat as previously described. Trunnion 172 may be of Delrin. Between the ends of the trunnion 172 and sleeves 38 are received depending limbs of an oscillating cradle 174 that carries an unidirectional roller clutch assembly generally indicated by reference numeral 176. Independent movement of the cradle 174 and the trunnion 172 is prevented by a posterior stop screw or stud 178 upstanding from the trunnion 172. The clutch assembly includes an anterior wall defining plate 170 a space that is downwardly convergent in side profile and medical and lateral walls between which is secured a stout backing plate 184 over which the plate 170 travels but which resists load thereon in posterior direction. A roller 186 in a cage 188 fits into the convergent space with its axis in a medial/lateral direction and is biased towards disengagement with the convergent surfaces of wall 180 and plate 70 by a coil spring 190 in compression between a lower face of cage 188 and the top face of trunnion 172 the coil spring being stabilised by a pin 191 upstanding from an anterior position on trunnion 172 that is slidingly received in a lower end face of cage 188. A second coil spring 194 in a guide 196 attached between the tops of medial and lateral walls 182 is in compression between an upper face of cage 188 and a loading button 198 slideably supported in and projecting from the guide 196. The balance of compressive loads in springs 190 and 194 is such that the roller 186 is disengaged until the leaf spring 164 applies load thereto, the stiffness of the coil spring 194 being significantly higher than that of the spring 190. Accordingly when weight is applied with knee unflexed as in the stance phase of walking the roller 186 is jammed between the convergent surfaces, preventing further flexion, whilst extension is possible because upward movement of extension rod 166 and plate 170 is not impeded by the roller even with weight applied. The roller 186 and convergent surfaces therefore act as a unidirectional clutch operating to prevent flexion but not extension when weight is applied to the knee.

A thin stiff buffer 200 controls the extended position of upper member 150 and platform 152 while a larger buffer 202 collapses when load is applied to the platform 152 and allows platform 152 to rotate clockwise relative to upper member 30. The larger buffer 202 controls the characteristic of a so-called "bouncy feature". The upper member and platform assembly could be used with a locked knee to provide the bouncy feature when walking with a stiff leg as is often required by older patients. When the limb is unloaded the leaf spring or pawl 164 is in an upper position and has a profile which is a radius about the knee pivot 14. When the limb is loaded the clockwise rotation of platform 152 relative to upper member 150 causes the leaf spring 164 to move to a lower position.

The actual stance control pivots about the axis of the trunnion 172 and comprises a roller 156, cage 180 and blade 170 which is part of the extension rod 166. When the roller 156 is in an upper position the blade 170 is free to move vertically in either direction. When the roller 156 is forced into a lower position it engages with the cage 180 and glade 170 causing the blade 170 to be locked between the roller 156 and a friction pad 184. In this position the knee cannot flex but can be extended. Any increase in flexing load causes an increase in resisting force due to the geometry of the roller 156, the blade 170 and the inclined plane 180 at the front of the cage. The friction pad material is selected to provide a high friction level and thus reduce the load on the roller 156.

Now the vertical position of the roller 156 is determined by the position of the last spring 164 such that when the limb is loaded the spring 164 is in its lower position and the roller 156 is in its lower position and the limb will not flex further. When the limb is unloaded the position is reversed. A strut between the spring 164 and the roller is of variable length to allow adjustment. It also incorporates a dead length spring so that the spring 164 may continue to move a platform 152 rotates relative to upper member 150 after the roller 156 is engaged, and the desired bouncy feature is obtained.

The stance phase control is intended to work over a shin angle range used when walking normally typically up to 30°C. This angle is controlled by the dimension D.

The angular position of the cradle 174 is determined by the extension rod 166 of which the blade is part. The spring 190 mounted on the cradle 174 forces the roller 186 upwards when not in use. The spring 190 is also intended to rotate the cage 180 clockwise about the trunnon centre so that the blade 170 runs free of the roller 156 and the friction pad 184. The actual cage position when not in use is determined by adjusting screw 178.

## Claims

1. A knee prosthesis comprising a top hinge member (10), a bottom hinge member (12) having spaced cheek plates (36) facing in a posterior direction, pivot means (14) interconnecting the top and bottom hinge member (10, 12), control rod means (22) depending from and pivoted at an upper end to the top hinge member (10), oscillating trunnion means (30) pivoted between the cheek plates (36) and having a transverse aperture (46) in which the control rod means (22) is slideably received, and means (54, 56) operatively connecting the control rod means (22) and the trunnion means (30) to arrest relative movement between the top and bottom hinge member (10,12).

2. A prosthesis according to Claim 1, wherein resilient buffer means (56) on a portion (28) of the control rod means (22) depending beneath the trunnion means (30) encounters an abutment (54) on the underside of the trunnion means (30) to define an extended relative position of the top and bottom hinge members (10,12), adjustable retaining means (60) on the underside of the resilient buffer means (56) permits the position of the buffer means (56) along the control rod means (22) to be set to define a desired extended relative position of the top and bottom hinge members (10, 12), the resilient buffer means (56) is an annular washer on the control rod means (22) and the adjustable retaining means is an adjustable nut (60) and a locknut (64) on a threaded lower region of the control rod means, and wherein bearing sleeve means (48) for the control rod means (22) fits into the transverse aperture (46).

3. A prosthesis according to Claim 1 or 2, wherein flanged bearing inserts (38) for the trunnion means (30) are inserted from inner faces of the cheek plates (36) with ends of a trunnion body (30) in sliding contact with flanges (38) of the inserts, and stub axles (40) inserted through said bearing inserts (38) into recesses (34) in the ends of the trunion body (30) are retained by threaded fasteners (42) received in axial bores (32) of the trunnion body (30).

4. A prosthesis according to any preceding claim, wherein the control rod means (22) is formed with a collar (26) located between its pivoted upper end (24) and the trunnion means (30) and a coil spring (52) on the control rod means (22) and held in compression between the collar (26) and the trunnion means (30) urges the top and bottom hinge members (10, 12) towards their extended positions at low angles of flexion, the pivoted upper end (24) of the control rod means (22) passing from posterior to anterior side of a line joining the knee pivot means (14) with the trunnion means (30) so that the top and bottom hinge members (10, 12) are urged to an extended position at high angles of flexion.

5. A prosthesis according any preceding claim, wherein knee lock means (90) operable to arrest relative movement of the top hinge member (10) and the bottom hinge member (12) at an unflexed attitude of the knee is operatively connected to the control rod means (70).

6. A prosthesis according to Claim 5, wherein the knee lock means is a latch bar (90) rotatable about a transverse axis to and from an operative position between a nose (80) on the control rod means (70) and a longitudinally slotted member (102) on the trunnion means (72), the latch bar (90) when in its operative position preventing the nose (80) from descending along the slot (110) so that the knee is held unflexed.

7. A prosthesis according to Claim 6, wherein a packing piece (112, 114) of adjustable length is provided between the slotted member ((102) and the trunnion means (72), and the locking bar (90) is attached to a carrier (76) that fits onto an anterior face (77) of and is pivoted to the control rod means (70).

8. A knee prosthesis according to any preceding claim, wherein damper means is operably connected to the control rod means (70) to control extension of the knee during the swing phase of walking.

9. A prosthesis according to Claim 8, wherein the damper means is arranged to offer minimal resistance during flexion of the knee.

10. A knee prosthesis according to Claim 9, wherein the damper means comprises a cylinder (120), an annular piston (130) in the cylinder, tubular piston rod means (126) in the cylinder (120) that passes through the piston (136) and through a lower end of the cylinder (120) to contact abutment means (56) at a lower end of the rod means (70) and movable therewith, means (130) urging the piston rod means (126) against the abutment means (56), lost motion connector means (134, 138) interconnecting the piston rod means (70) and the piston (136) so that when the piston rod (70) descends as the knee flexes the piston (136) is spaced from a collar (134) on the rod means (70) and fluid can flow directly from the piston (136) to the rod side of the cylinder (120) and when the piston rod means (70) ascends as the knee unflexes direct fluid flow from the rod to the piston side of the cylinder (120) is prevented, by-pass passage means (140) including a flow restricting orifice (142) interconnecting rod and piston sides of the cylinder (120) to provide a fluid return path.

11. A prosthesis according to Claim 10, wherein the size means (126) against the abutment means (56), lost motion connector means (134, 138) interconnecting the piston rod means (70) and the piston (136) so that when the piston rod (70) descends as the knee flexes the piston (136) is spaced from a collar (134) on the rod means (70) and fluid can flow directly from the piston (136) to the rod side of the cylinder (120) and when the piston rod means (70) ascends as the knee unflexes direct fluid flow from the rod to the piston side of the cylinder (120) is prevented, by-pass passage means (140) including a flow restricting orifice (142) interconnecting rod and piston sides of the cylinder (120) to provide a fluid return path.

11. A prosthesis according to Claim 10, wherein the size of the orifice (142) is variable and a branch passage (144) connects the by-pass passage (140) to a seal (146) to the piston rod (70) so that pressure in the by-pass passage (140) remains low and the seal (146) is lubricated.

12. A knee prosthesis according to any preceding claim, wherein means in the top hinge member (150) acting on the control rod means (166) locks the position of the control rod means (166) when the weight of the user is applied to the leg thereby arresting flexion or extension of the knee.

13. A prosthesis according to Claim 12, wherein a platform (152) for connection to upper parts of the limb is hinged to the top hinge member for rotation about a transverse axis, an actuating member (164) attached to the platform applying clutch means in the lower hinge member (12) when the weight of the user is applied.

14. A prosthesis according to Claim 13, wherein the actuating member (164) is a leaf spring, the clutch is a roller (186) having a transversely directed axis captive between an anterior wall (180) on the trunnion means (172) and a sliding plate (170) defined by a transversely enlarged region of the control rod means (166), said plate (170) and said anterior wall (180) defining between them a space of downwardly convergent profile in side view, the roller (186) is supported in cage means (188) connected by a spring (194) in compression to a plunger (198) arranged to be depressed by the actuating member (164) as weight is applied to the platform (152) and means is provided on the trunnion (172) for biasing the roller (186) out of engagement with said convergent surfaces (170, 180), said means being a coil spring (190) held in compression between said trunnion (172) and said cage means (188).

15. A prosthesis according to Claim 14, wherein said sliding plate (170) travels over a backing plate (184) supported by lateral walls 9182) extending from the anterior wall (180), said backing plate (184) being of high friction material to reduce load on the roller (186), said trunnion means is in two parts (172, 174), one of which is a cradle (174) that supports the anterior and lateral walls and the backing plates and has bifurcated legs between which an inner part (172) fits through which the lowermost part of the control rod means (166) slides.

FIG.1.

FIG.2.

FIG. 3.

FIG. 4.

FIG. 5.

FIG. 6.

FIG. 7.

FIG. 8.

FIG.9.

FIG.10.

FIG.11.

FIG.12.

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| A | US-A-4 065 815  (CHEN SEN-JUNG) * figures 1,2; claim 1 * | 1 | A 61 F  2/64 |
| A | US-A-3 407 409  (PRAHL) * figures 1,2;  column 3, lines 18-34 * | 1 | |
| A | DE-U-6 603 485  (KLEYLEIN) * figure 1; page 5, lines 16-20 * | 1 | |
| A | FR-A-1 565 589  (DANGER) * figures 1,2 * | 1 | |

----

| TECHNICAL FIELDS SEARCHED (Int. Cl.4) |
|---|
| A 61 F  2/00 |

The present search report has been drawn up for all claims

| Place of search | Date of completion of the search | Examiner |
|---|---|---|
| BERLIN | 26-10-1987 | KANAL P K |